Europäisches Patentamt

European Patent Office

Office européen des brevets

(11) Veröffentlichungsnummer: **0 033 497**
**B1**

(12) **EUROPÄISCHE PATENTSCHRIFT**

(45) Veröffentlichungstag der Patentschrift: **30.01.85**

(21) Anmeldenummer: **81100502.4**

(22) Anmeldetag: **23.01.81**

(51) Int. Cl.⁴: **A 61 B 6/00**

(54) **Stativ für ein unter der Patientenlagerungsplatte eines Röntgenuntersuchungsgerätes einschiebbares röntgenographisches Bilderfassungsgerät.**

(30) Priorität: **04.02.80 DE 3003976**

(43) Veröffentlichungstag der Anmeldung:
**12.08.81 Patentblatt 81/32**

(45) Bekanntmachung des Hinweises auf die
Patenterteilung:
**30.01.85 Patentblatt 85/05**

(84) Benannte Vertragsstaaten:
**BE DE FR SE**

(56) Entgegenhaltungen:
**DE-A-1 548 520**
**DE-C- 586 827**
**FR-A-2 351 638**
**US-A-2 369 507**

(73) Patentinhaber: **Siemens Aktiengesellschaft**
**Berlin und München Wittelsbacherplatz 2**
**D-8000 München 2 (DE)**

(72) Erfinder: **Dornheim, Hans-Peter**
**Meisenweg 15**
**D-8521 Bubenreuth (DE)**

· Courier Press, Leamington Spa, England.

## Beschreibung

Die Erfindung bezieht sich auf ein Stativ für ein unter einer Patientenlagerungsplatte eines Röntgenuntersuchungsgerätes einschiebbares röntgenographisches Bilderfassungsgerät mit einem am Fußboden zweidimensional verfahrbaren Stativwagen, einem am Stativwagen höhenverstellbaren Hubwagen und einer am Hubwagen um eine horizontale Achse um 90° schwenkbaren Halterung für das aus einer Lage mit horizontaler Bildschicht in eine solche mit vertikaler Bildschicht kippbare Bilderfassungsgerät.

Die DE—AS 25 57 810 offenbart ein Stativ für ein röntgenographisches Bilderfassungsgerät, das aus einer auf dem Fußboden zweidimensional verfahrbaren Stativsäule, einem an der Stativsäule höhenverstellbaren Hubwagen und zwei an diesem Hubwagen um eine horizontale Achse schwenkbaren Auslegern besteht. Am Ende dieser beiden Ausleger ist eine Tragplatte für ein aufzuschraubendes Bilderfassungsgerät um eine weitere horizontale, zur erstgenannten horizontalen Achse parallele Achse schwenkbar gelagert. Mit diesem Stativ kann das auf der Tragplatte aufgeschraubte Bilderfassungsgerät bei abgesenktem Hubwagen, abgesenkten Auslegern und horizontal gestellter Tragplatte unter die Patientenlagerungsplatte eines Röntgenuntersuchungsgerätes eingeschoben werden, um in Verbindung mit einer Obertrisch-Röntgenröhre Aufnahmen mit vertikaler Strahlenrichtung vornehmen zu können. Will man mit dem Bilderfassungsgerät Aufnahmen mit horizontaler Strahlenrichtung vornehmen, so muß das Stativ unter der Patientenlagerungsplatte hervorgezogen, muß der Hubwagen am Stativ hochgeschoben, müssen die beiden Ausleger um einen vorgegebenen Winkel hochgeschwenkt werden und muß dann die Tragplatte mit dem Bilderfassungsgerät um ihre horizontale Achse entgegengesetzt zu den Auslegern, jedoch um den gleichen Winkel, gedreht werden. Erst dann kann das Stativ für das röntgenographische Bilderfassungsgerät wieder an die Patientenlagerungsplatte herangeschoben und wieder zum Röntgenuntersuchungsgerät bzw. der Röntgenröhre justiert werden. Danach muß der Hubwagen so weit abgesenkt werden, bis das Bilderfassungsgerät unmittelbar über der Patientenlagerungsplatte steht. Bei dieser Konstruktion ist der erhebliche Bedienungsaufwand störend. Darüber hinaus ist es eine Eigenart dieses Gerätes, daß die Stativsäule wegen ihrer Höhe stets seitlich der Patientenlagerungsplatte angeordnet werden muß und daher bei der Untersuchung des Patienten im Wege steht. Auch ist der konstruktive Aufwand, der bei diesem Stativ getrieben wird, beträchtlich.

Der Erfindung liegt die Aufgabe zugrunde, ein Stativ der eingangs genannten Art derart auszubilden, daß die Untersuchung des Patienten möglichst wenig behindert wird und das Stativ sich einfacher handhaben läßt. Darüber hinaus sollte eine einfachere und preiswertere Konstruktion gesucht werden.

Die Aufgabe wird erfindungsgemäß durch die im Kennzeichen des Patentanspruchs 1 angegebenen Merkmale gelöst. Dadurch läßt sich das Stativ mitsamt dem daran befestigten Bilderfassungsgerät bei der häufigsten Verwendungsart, nämlich bei der Untersuchung mit vertikalem Strahlengang, vollständig unter die Patientenlagerungsplatte des Röntgenuntersuchungsgerätes fahren und stört daher bei der Untersuchung überhaupt nicht mehr. Darüber hinaus erfordert diese Bauweise beim Übergang von einer Untersuchungstechnik mit vertikalem zu einer solchen mit horizontalem Strahlengang nur noch eine einzige Höhenverstellung und eine einzige Schwenkbewegung. Schließlich erlauben die Kupplungsmittel eine definierte und leicht reproduzierbare Positionierung des Bilderfassungsgerätes. Aufwendige Zentrierungen zum Röntgenuntersuchungsgerät bzw. zur Röntgenröhre erübrigen sich somit.

Wird der Hubwagen beim Übergang von der Untersuchung mit vertikalem Strahlengang zur Untersuchung mit horizontalem Strahlengang schräg hochgeschoben, so entfernt er sich dabei auch in Querrichtung von der Patientenlagerungsplatte. Das hat zur Folge, daß der Stativwagen bei einer gegebenen Position des Bilderfassungsgerätes über der Patientenlagerungsplatte tiefer unter der Patientenlagerungsplatte steht und somit weniger stört. Außerdem ist mit dieser Maßnahme erreicht worden, daß das Stativ mitsamt dem vertikal gestellten Bilderfassungsgerät weiter zur Mitte der Patientenlagerungsplatte hin eingeschoben werden kann, was insbesondere bei Untersuchungen des Schädels oder der Extremitäten von großem Vorteil sein kann.

Die Handhabung des Stativs wird wesentlich erleichtert, wenn in vorteilhafter Weiterbildung der Erfindung das Gesamtgewicht des Hubwagens, der Halterung und des Bilderfassungsgerätes durch mindestens eine im Stativwagen eingebaute Stützfeder kompensiert ist. In solchen Fällen brauchen nur geringfügige Kräfte ausgeübt zu werden, um den Wechsel von der Betriebsart mit horizontalem zu jener mit vertikalem Strahlengang vorzunehmen.

Die Handhabung des Stativs kann noch weiter vereinfacht werden, wenn in zweckmäßiger Weiterbildung der Erfindung an die Höhenlage der Auflagefläche und der Unterseite der Patientenlagerungsplatte angepaßte Anschläge für die obere und die untere Begrenzung des Hubweges des Hubwagens vorgesehen sind. Solche Anschläge ermöglichen es, den Hubwagen beim Absenken auf Anhieb und ohne jede Justierung in die Position zu bringen, in der er mit dem geringstmöglichen Abstand unter der Patientenlagerungsplatte einzuschieben ist bzw. beim Hochziehen über der Patientenlagerungsplatte steht.

Eine weitere Vereinfachung der Handhabung des Stativs wird erreicht, wenn in vorteilhafter Weiterbildung der Erfindung an seinen beiden Längsseiten Führungslappen angeordnet sind, die in entsprechende, quer zur Längsachse der Patientenlagerungsplatte ausgerichtete Profilschienen des Röntgenuntersuchungsgerätes einschiebbar sind. Bei der Verwendung eines zweidimensional verschiebbaren Stativwagens wird so die Zentrierung auch dann beibehalten, wenn das Bauelement des Röntgenuntersuchungsgerätes, an das angekuppelt wird, sich bei der Untersuchung verschiebt, wie das z.B. bei Zielgeräten oder Patientenlagerungsplatten der Fall ist.

Weitere Einzelheiten der Erfindung werden anhand eines in den Figuren dargestellten Ausführungsbeispieles näher erläutert. Es zeigen:

Fig. 1 eine schaubildliche Darstellung eines Röntgenuntersuchungsgerätes mit einem unter der Patientenlagerungsplatte eingefahrenen Stativ für ein röntgenographisches Bilderfassungsgerät in Vorderansicht,

Fig. 2 eine schaubildliche Darstellung des Röntgenuntersuchungsgerätes und des Stativs der Figur 1 in Seitenansicht,

Fig. 3 eine teilweise aufgebrochene Seitenansicht des Stativs, und

Fig. 4 eine Ausschnittvergrößerung der Ankuppelstelle zwischen dem Stativ und dem Röntgenuntersuchungsgerät in der Figur 1.

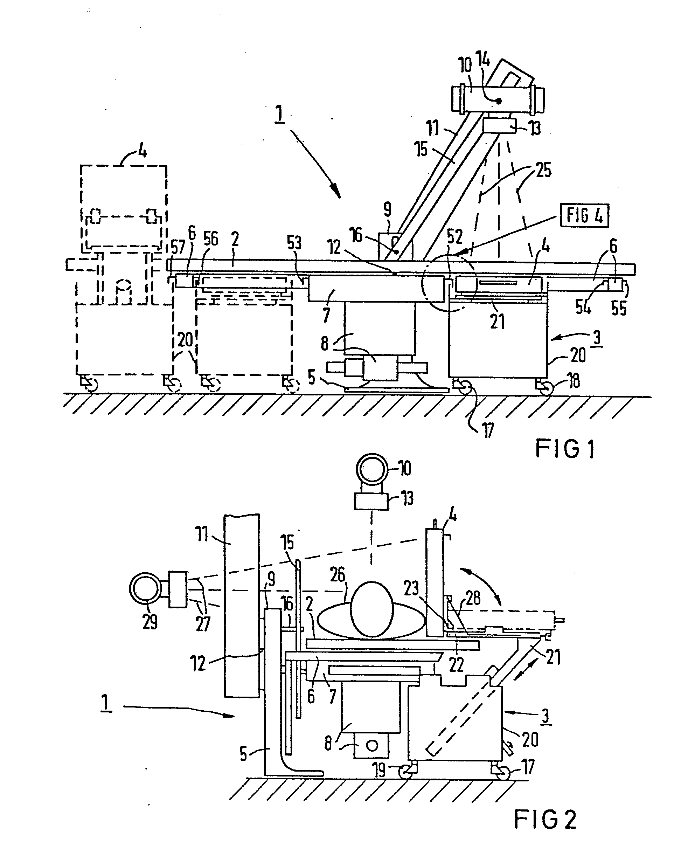

In der Figur 1 erkennt man ein Röntgenuntersuchungsgerät 1, bei dem unter der horizontal gestellten Patientenlagerungsplatte 2 ein Stativ 3 für ein röntgenographisches Bilderfassungsgerät 4 eingeschoben ist. Das Röntgenuntersuchungsgerät 1 besteht aus einem auf dem Fußboden ruhenden Fußgestell 5, einem am Fußgestell in hier nicht weiter dargestellter Weise um eine horizontale Achse kippbar gelagerten Tischrahmen 6, auf dem die Patientenlagerungsplatte 2 längs- und querverschiebbar gelagert ist. Unter der Patientenlagerungsplatte 2 ist ein Zielgerät 7 mit einer Bildverstärker-Fernseheinrichtung 8 am Tischrahmen 6 längsverschiebbar gelagert. Das Fußgestell 5 trägt einen Schichtblock 9, an der der die Röntgenröhre 10 tragende Röhrenturm 11 um eine weitere horizontale Achse 12 schwenkbar gelagert ist. Die Röntgenröhre ist mitsamt der angeflanschten Tiefenblende 13 am Röhrenturm um eine weitere horizontale Achse 14 schwenkbar gelagert. Diese letztgenannte horizontale Achse 14 ist parallel zu der Achse 12 ausgerichtet, um die der Röhrenturm 11 am Schichtbock 9 schwenkbar gelagert ist. Die Röntgenröhre kann bei Bedarf mit einer Schichtstange 15 gekuppelt werden, die ihrerseits um eine am Schichtbock 9 höhenverstellbare Schichtachse 16 schwenkbar gelagert und an ihrem gegenüberliegenden Ende mit dem Zielgerät 7 gekuppelt ist.

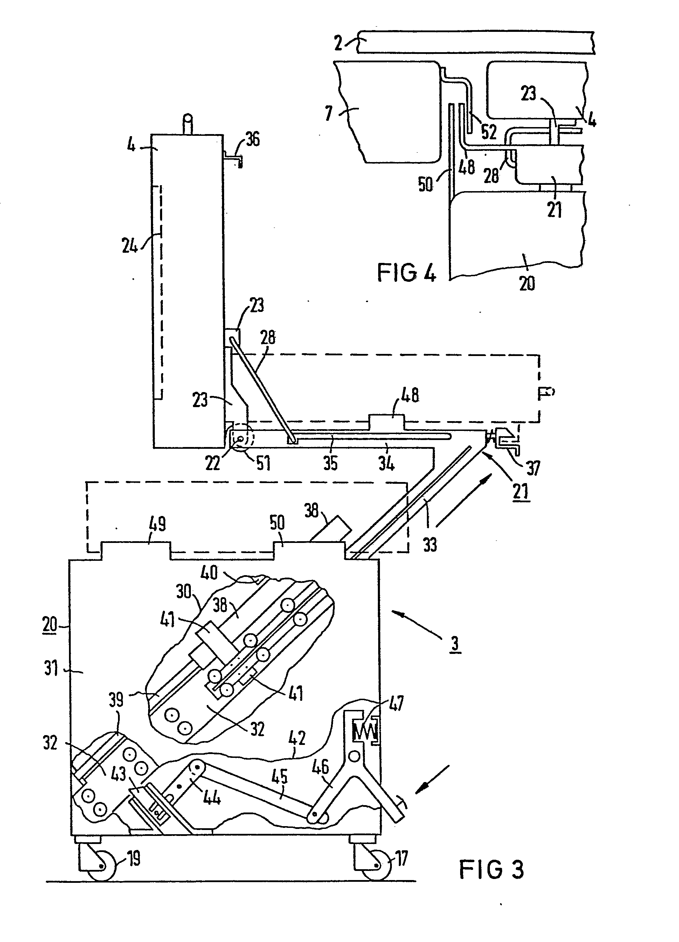

Unter der patientenlagerungsplatte 2 erkennt man das Stativ 3 für das röntgenographische Bilderfassungsgerät 4. Dieses besitzt einen auf dem Fußboden auf schwenkbaren Rollen 17, 18, 19 zweidimensional verfahrbaren kastenförmigen Stativwagen 20, in dem ein Hubwagen 21, der eine um eine horizontale Achse 22 um 90° schwenkbare Halterung 23 trägt, höhenverstellbar geführt ist. An der Halterung ist das Bilderfassungsgerät 4 befestigt. Es läßt sich, wie später anhand der Figuren 2 und 3 gezeigt wird, mitsamt der Halterung 23 von einer Position mit horizontal ausgerichteter in eine solche mit vertikal ausgerichteter Bildschicht 24 schwenken. In der Figur 1 sind gestrichelt weitere mögliche Arbeitspositionen für das Stativ angedeutet.

Die Figur 2 zeigt das Röntgenuntersuchungsgerät 1 der Figur 1 von der Stirnseite her. Das Bilderfassungsgerät 4, das in der Darstellung der Figur 1 zur Untersuchung mit senkrechter Röntgenstrahlung 25 vollständig unter der Patientenlagerungsplatte 2 heruntergefahren war, ist in der Figur 2 zur Untersuchung eines auf der Patientenlagerungsplatte 2 liegenden Patienten 26 mit horizontaler Röntgenstrahlung 27 nur teilweise unter der Patientenlagerungsplatte geschoben. Dafür ist der Hubwagen 21 des Stativs 3 in seiner obersten Stellung herausgezogen dargestellt. Außerdem ist das Bilderfassungsgerät 4 in der Figur 2 samt Halterung 23 hochgeschwenkt und mit einem Haltebügel 28 am Hubrahmen 21 arretiert. In dieser Position des Bilderfassungsgerätes 4 kann ein auf der Patientenlagerungsplatte 2 liegender Patient 26 mit Hilfe einer zweiten, gegenüber dem Bilderfassungsgerät angeordneten Röntgenröhre 29 mit horizontal ausgerichteter Röntgenstrahlung 27 untersucht werden.

Weitere Einzelheiten des Aufbaues des Stativs 3 für das röntgenographische Bilderfassungsgerät 4 sind aus der vergrößerten Darstellung der Figur 3 zu erkennen. Diese Figur läßt durch den Durchbruch 30 in der Seitenwand 31 des Stativwagens 20 eine der beiden an den beiden einander gegenüberliegenden Seiten des Stativwagens 20 auf der Innenseite angebrachten Röllchenbahnen 32 (nur eine dargestellt) erkennen. Zwischen diesen ist der Hubwagen 21 mit seinem unteren aus T-Profilen bestehenden Rahmenteil 33 (nur eines dargestellt) verschiebbar. Die Röllchenbahnen 32 sind so angeordnet, daß sich der Hubwagen 21 längs einer um 45° gegenüber der Horizontalen geneigten Ebene in der Höhe verschieben läßt. Das in den Röllchenbahnen geführt Rahmenteil 33 des Hubwagens 21 ist mit einer horizontalen Plattform 34 verschweißt. An der dem schrägen Rahmenteil abgewandten oberen Kante der rahmenförmigen Plattform 34 des Hubwagens 21 ist die Halterung 23, die ihrerseits wiederum mit dem röntgenographischen Bilderfassungsgerät 4 verschraubt ist, um die parallel zu dieser Kante ausgerichtet Achse 22 schwenkbar gelagert.

Das röntgenographische Bilderfassungsgerät 4 läßt sich daher aus einer in der Figur 3 gestrichelt angedeuteten Position, bei der die Bildschicht 24 horizontal ausgerichtet ist, in die in der Figur 3 ausgezogen dargestellte, um 90° geschwenkte Position kippen, in der die Bildschicht senkrecht steht.

In der ausgezogenen, senkrechten Position läßt sich das röntgenographische Bilderfassungsgerät über einen an der Halterung 23 drehbar gelagerten und mit seinen beiden freien Enden in je einer Nut 35 (nur eine dargestellt) der Plattform 34 des Hubwagens 21 geführten Haltebügel 28 arretieren. In der horizontalen Position rastet das röntgenographische Bilderfassungsgerät 4 mit einem Profilteil 36 hinter eine in der Plattform 34 des Hubwagens 21 eingelassene federbelastete Klinke 37 ein. An dem längs der Röllchenbahnen 32 im Stativwagen 20 verschiebbaren unteren Rahmenteil 33 des Hubwagens 21 ist eine Gasfeder 38 befestigt. Deren Kolbenstange 39 stützt sich im Stativwagen ab. Im Durchbruch 30 in der Seitenwand 31 des Stativwagens 20 erkennt man auch einen oberen Anschlag 40. Dieser ist mit der Halterung 41 der Gasfeder 38 in der obersten Stellung des Hubwagens 21 in Eingriff. Die Gasfeder 38 ist so dimensioniert, daß sie das Gewicht des Hubwagens 21 nebst Halterung 23 und Bilderfassungsgerät 4 etwas überkompensiert. Durch die Gasfeder wird der Hubwagen daher gegen den oben Anschlag gepreßt. Dieser Anschlag 40 ist in hier nicht weiter dargestellter Weise verstellbar ausgebildet und an die jeweilige Höhenlage der Patientenlagerungsplatte 2 des Röntgenuntersuchungsgerätes 1 angepaßt. Im unteren Bereich der Röllchenbahnen 32 erkennt man hinter einem weiteren Durchbruch 42 in der Seitenwand 31 des Stativwagens einen verstellbaren Anschlag 43, der in der untersten Stellung des Hubwagens 21 mit der Halterung 41 für die Gasfeder 38 in Eingriff bringbar ist. Dieser Anschlag wird über einen gabelförmig geschlitzten zweiarmigen Hebel 44 und eine Schubstange 45 von einem Fußpedal 46 betätigt. Der untere Anschlag 43 wird von einer Druckfeder 47 gegen die Halterung 41 der Gasfeder 38 gedrückt.

In der Figur 3 erkennt man deutlich einen der zu beiden Seiten der rahmenförmigen Plattform 34 des Hubwagens 21 aufragenden Führungslappen 48. Auch an den beiden Seitenwänden 31 des Stativwagens 20 ragen je zwei Führungslappen 49, 50 für die Kupplung des Bilderfassungsgerätes 4 mit dem Röntgenuntersuchungsgerät 1 auf. Die beiden Führungslappen 49, 50 auf jeder der beiden Seitenwände des Stativwagens befinden sich in der Ebene der zugehörigen Seitenwand 31. Die Führungslappen 48 (nur einer sichtbar) auf den beiden Seiten der Plattform 34 des Hubwagens 21 sind seitlich so weit herausgeführt, daß sie mit den Führungslappen 49, 50 auf den Seitenwänden 31 des Stativwagens fluchten und sich bei abgesenkter Plattform exakt zwischen den beiden Lappen der jeweiligen Seitenwand des Stativwagens befinden. In der Figur 3 ist auch die um die Achse 22 geschlungene Torsionsfeder 51 zu erkennen, durch die das Drehmoment des Bilderfassungsgerätes 4 weitgehend kompensiert ist.

In der Darstellung der Figur 4, die eine Ausschnittvergrößerung der Kuppelstelle des Stativwagens 20 mit dem Zielgerät 7 zeigt, fluchten die Führungslappen 48, 49, 50 des Stativwagens und des Hubwagens 21 nicht ganz genau. Dies geschah aus Gründen der Übersichtlichkeit. So kann man in der Figur 4 den Führungslappen 48, der seitlich am Hubwagen vorsteht, von den Führungslappen 49, 50 des Stativwagens 20 besser unterscheiden. Die Figur 4 zeigt, wie die hintereinanderliegenden Führungslappen der Seitenwand des Stativwagens und des Hubwagens hinter einem L-förmigen Profilteil 52 an der Seite des Zielgerätes 7 des Röntgenuntersuchungsgerätes 1 eingreifen. Weitere Profilteile 53, 54, 55, 56, 57 befinden sich, wie die Figur 1 zeigt, auf der anderen Seite des Zielgerätes 7 und an den beiden äußeren Holmen des Tischrahmens 6, und zwar jeweils auf deren Innen- und Außenseite.

Bei der Untersuchung eines auf der Patientenlagerungsplatte 2 des Röntgenuntersuchungsgerätes 1 liegenden Patienten 26 kann nunmehr das Stativ 3 mit dem röntgenographischen Bilderfassungsgerät 4 bei Bedarf unter die Patientenlagerungsplatte 2 des Röntgenuntersuchungsgerätes unmittelbar neben das Zielgerät 7 so eingefahren werden, daß die Führungslappen 49, 50 des Stativwagens als auch der Führungslappen · 48 der Plattform 34 des Hubwagens 21 hinter das entsprechende L-förmige Profilteil 52, 53 des Zielgerätes 7 eingreifen. In dieser Position befindet sich die Bildschicht 24 unabhängig von der Schwenklage des Röhrenturmes 11 in einem definierten Abstand vom Zielgerät. Das bedeutet, daß im weiteren Untersuchungsablauf keine Rücksicht mehr auf die Lage des röntgenographischen Bilderfassungsgerätes 4 genommen zu werden braucht, weil dieses den Bewegungen des Zielgerätes 7, wenn letzteres durch Schwenken des Röhrenturmes von der Schichtstange 15 mitgenommen wird, folgt. Beim Wechsel vom Untersuchungsbetrieb mit dem Zielgerät 7 zum Aufnahmebetrieb mit dem röntgenographischen Bilderfassungsgerät 4 sind daher ausgehend von der Position mit senkrechtem Röhrenturm 11, unabhängig von den zwischendurch vorgenommenen Untersuchungen, immer genau dieselben vom Röntgenuntersuchungsgerät automatisch anfahrbaren Schwenkwinkel des Röhrenturmes 11 um die horizontale Achse 12 und der Röntgenröhre 10 um die horizontale Achse 14 erforderlich, um den Strahlengang 25 vom Zielgerät 7 weg auf das röntgenographische Bilderfassungsgerät 4 und wieder zurück zu zentrieren. Zugleich ist bei diesem Wechsel der Untersuchungsart die Verschiebung des Patienten 26 kleiner als jene bei feststehendem Bilderfassungsgerät 4, weil ja das Zielgerät 7 mit dem Bilderfassungsgerät 4 beim Schwenken des Röhrenturmes 11 der Röntgenröhre 10 entgegenkommt.

Da die Führungslappen 49, 50 zu beiden Seiten der Plattform 34 und des Stativwagens 20 und die Profilteile 52 bis 57 zu beiden Seiten des Zielgerätes 7 und des Tischrahmens 6 und zwar jeweils an der Innen- wie an der Außenseite des Holms angebracht sind, ist es sowohl möglich, das Stativ mit dem röntgenographischen Bilderfassungsgerät zu beiden Seiten des Zielgerätes als auch zu beiden Seiten des Tischrahmens 6 und zwar jeweils sowohl auf der Innenseite des Tischrahmens als auch auf der Außenseite des Tischrahmens, wie das gestrichelt in der Figur 1 angedeutet ist, anzukuppeln. In diesen Fällen ist gewährleistet, daß die Position des Bilderfassungsgerätes 4 unabhängig von der Verschiebung des Zielgerätes 7 bei der Verschwenkung des Röhrenturmes 11 stets zum Röntgenuntersuchungsgerät 1 positioniert ist. Insbesondere bei Untersuchungen des Schädels sind die in der Figur 1 links gestrichelt angedeuteten Positionen des Stativs für das Bilderfassungsgerät von Bedeutung. In diesem Fall kann die Patientenlagerungsplatte 2 in Längsrichtung über bzw. unter das Bilderfassungsgerät 4 geschoben werden. In allen gezeigten Positionen des Bilderfassungsgerätes kann der Stativwagen 20 infolge seiner geringen Bauhöhe vollständig unter die Patientenlagerungsplatte 2 eingefahren werden. Er behindert somit in keiner Weise die Untersuchung des Patienten.

Soll eine Untersuchung mit horizontalem Strahlengang vorgenommen werden, so genügt es, das Stativ 3 für das Bilderfassungsgerät 4 unter der Patientenlagerungsplatte hervorzuziehen, den unteren Anschlag 43 durch Druck auf das Fußpedal 46 über die Schubstange 45 und den zweiarmigen Hebel 44 entgegen der Kraft der Druckfeder 47 zurückzuziehen, um der Gasfeder 38 Gelegenheit zu geben, den Hubwagen an den oberen Anschlag 40 anzudrükken. In dieser in der Figur 3 dargestellten Position des Hubwagens 21 kann das röntgenographische Bilderfassungsgerät 4 durch Niederdrücken der federbelasteten Klinke 37 aus ihrer gestrichelt angedeuteten horizontalen Position in die ausgezogen dargestellte vertikale Position gekippt und durch Einrasten des Haltebügels 28 in der Nut 35 arretiert werden. In dieser Position läßt sich das Bilderfassungsrät, wie in der Figur 2 gezeigt ist, durch Einfahren des Stativwagens 20 unter die Patientenlagerungsplatte 2 beliebig weit über die Patientenlagerungsplatte schieben. Irgendwelche Justiermaßnahmen für die Höhe oder den Schwenkwinkel des Bilderfassungsgerätes sind hierbei nicht erforderlich. Infolge der schrägen Anordnung des zwischen den Röllchenbahnen 32 verschiebbaren Rahmenteils 33 behindert letzterer das Einschieben so gut wie gar nicht.

Anstelle der Verwendung einer Gasfeder lassen sich auch andere Federn oder auch Gegengewichte verwenden. Es wäre auch möglich, die Rollen am Hubwagen anzubringen und sie zwischen an den Seitenwänden des Stativwagens fest angebrachte Führungen laufen zu lassen. Schließlich ist es auch denkbar, den Hubwagen horizontal verschiebbar im Stativwagen zu lagern und die Halterung am Hubwagen senkrecht verschiebbar zu führen. Diese Konstruktion hätte aber den Nachteil, daß eine zusätzliche Stellbewegung beim Übergang von der Untersuchung mit vertikalem zu einer mit horizontalem Strahlengang erforderlich wäre. Auch in diesem Fall ließe sich aus den vorgenannten Gründen jede der Stellbewegungen durch Anschläge begrenzen.

**Patentansprüche**

1. Stativ (3) für ein unter einer Patientenlagerungsplatte (2) eines Röntgenuntersuchungsgerätes (1) einschiebbares röntgenographisches Bilderfassungsgerät (4), mit einem am Fußboden zweidimensional verfahrbaren Stativwagen (20), einem am Stativwagen (20) höhenverstellbaren Hubwagen (21) mit einer am Hubwagen (21) um eine horizontale Achse um 90° schwenkbaren Halterung (23) für das aus einer Lage mit horizontaler Bildschicht in eine solche mit vertikaler Bildschicht kippbare Bilderfassungsgerät (4), dadurch gekennzeichnet, daß eine gegenüber der Senkrechten geneigte Führungsbahn (32) an dem Stativwagen (20) angeordnet ist, daß der Hubwagen (21) eine horizontale Plattform (34) aufweist, an deren einem Ende das Bilderfassungsgerät (4) mittels den Halterung (23) schwenkbar gelagert ist, daß ein in der Führungsbahn (32) geführtes Rahmenteil (33) des Hubwagens (21) starr mit der Plattform (34) verbunden ist und mit ihr einen spitzen Winkel bildet, daß der Stativwagen (20), der am Stativwagen geführte Hubwagen (21) und die auf dem Hubwagen schwenkbar gelagert Halterung (23) in ihrer horizontalen Ausgangsstellung unterhalb des Bildererfassungsgerätes (4) angeordnet und daß mit dem Röntgenuntersuchungsgerät (1) in Eingriff bringbare Kuppelmittel (48, 49, 50) für die definierte Positionierung des Bilderfassungsgerätes (4) zum Röntgenuntersuchungsgerät (1) mit dem Stativwagen verbunden sind.

2. Stativ nach Anspruch 1, dadurch gekennzeichnet, daß das Gesamtgewicht des Hubwagens (21), der Halterung (23) und des Bilderfassungsgerätes (4) durch mindestens eine im Stativwagen (20) eingebaute Stützfeder (38) kompensiert ist.

3. Stativ nach Anspruch 1, dadurch gekennzeichnet, daß an die Höhenlage der Auflagefläche und der Unterseite der Patientenlagerungsplatte (2) angepaßte Anschläge (40, 43) für die obere und die untere Begrenzung des Hubweges des Hubwagens (21) vorgesehen sind.

4. Stativ nach Anspruch 1, dadurch gekennzeichnet, daß an seinen beiden Längsseiten Führungslappen (48, 49, 50) angeordnet sind, die in entsprechende, quer zur Längsachse der Patientenlagerungsplatte (2) ausgerichtete Profilschienen (52 bis 57) des Röntgenuntersuchungsgerätes (1) einschiebbar sind.

5. Stativ nach Anspruch 1, dadurch gekennzeichnet, daß die Kuppelmittel (48 bis 50) in an dem Untertischzielgerät (7) des Röntgenuntersuchungsgerätes (1) angebrachte Profilteile (52, 53) eingreifen.

6. Stativ nach Anspruch 1, dadurch gekennzeichnet, daß die Kuppelmittel (48 bis 50) in am Tischrahmen (6) der Patientenlagerungsplatte (2) angebrachte Profilteile (54 bis 57) eingreifen.

7. Stativ nach Anspruch 6, dadurch gekennzeichnet, daß die Kuppelmittel (48 bis 50) in an einer Stirnseite des Tischrahmens (6) angebrachte Profilteile (55, 57) eingreifen.

8. Stativ nach Anspruch 2, dadurch gekennzeichnet, daß die Stützfeder (38) das Gesamtgewicht des Hubwagens (21) und des daran befestigten Bilderfassungsgerätes (4) etwas überkompensiert und der Hubwagen über eine Klinke (43) in seiner untersten Stellung arretierbar ist.

9. Stativ nach Anspruch 1, dadurch gekennzeichnet, daß die Position des Bilderfassungsgerätes (4) sowohl in der Stellung mit horizontal als auch mit vertikal ausgerichteter Bildschicht (24) durch Anschläge (28, 35, 36, 37, 40, 41, 43) definiert ist.

10. Stativ nach Anspruch 1, dadurch gekennzeichnet, daß der um eine horizontale Achse (22) schwenkbaren Halterung (23) für die Kippung des Bilderfassungsgerätes (4) mindestens eine das Drehmoment kompensierende Feder (51) zugeordnet ist.

11. Stativ nach Anspruch 1, dadurch gekennzeichnet, daß die Führungsbahn (32) für den Hubwagen (21) 20° bis 70° gegenüber der Senkrechten geneigt ist.

**Revendications**

1. Statif (3) pour un appareil (4) d'enregistrement d'images radiographiques déplaçable en-dessous de la couchette pour patient (2) d'un appareil de radiodiagnostic (1), comportant un chariot porte-statif (20) déplaçable dans un plan au sol, un chariot de levage (21) réglable en hauteur sur le chariot porte-statif (20) et comportant un dispositif de fixation (23) pouvant pivoter de 90° autour d'un axe horizontal et prévu pour l'appareil d'enregistrement d'images (4) pouvant basculer depuis une position, dans lequelle la couche d'image est horizontale, dans une position dans lequelle la couche d'image est verticale, caractérisé par le fait qu'une glissière (32) inclinée par rapport à la verticale est disposée sur le chariot porte-statif (20), que le chariot de levage (21) comporte une plateforme horizontale (34) sur une extrémité de laquelle l'appareil d'enregistrement d'images (4) est monté de façon à pouvoir pivoter au moyen du dispositif de fixation (23), qu'une partie formant cadre (33), guidée dans la glissière (32), du chariot de levage (21), est reliée rigidement à la plateforme (34) et fait avec elle un angle aigu, que le chariot porte-statif (20), le chariot de levage (21) guidé sur le chariot porte-statif et le dispositif de fixation (23) monté de façon à pouvoir pivoter sur le chariot de levage sont disposées dans leur position de départ horizontale en-dessous de l'appareil d'enregistrement d'images (4), et que des moyens d'accouplement (48, 49, 50), qui peuvent être amenés en prise avec l'appareil de radiodiagnostic (1) et qui sont prévus pour le positionnement défini de l'appareil d'enregistrement d'images (4) par rapport à l'appareil de radiognostic (1), sont reliés au chariot porte-statif.

2. Statif suivant la revendication 1, caractérisé par le fait que le poids total du chariot de levage (21), du dispositif de fixation (23) et de l'appareil d'enregistrement d'images (4) est compensé par au moins un ressort de soutien (38) monté dans le chariot porte-statif (20).

3. Statif suivant la revendication 1, caractérisé par le fait que des butées (40, 43), qui sont adaptées à la position en hauteur de la surface d'appui et de la face inférieure de la couchette pour patient (2), sont prévues pour la limitation supérieure et la limitation inférieure de la course de levage du chariot de levage (21).

4. Statif suivant la revendication 1, caractérisé par le fait que sur ses deux côtés longitudinaux se trouvent disposées des languettes d'accouplement (48 à 50) qui peuvent être insérées dans des rails profilés correspondant (52 à 57) de l'appareil de radiodiagnostic (1), orientées transversalement par rapport à l'axe longitudinal de la couchette pour patient (2).

5. Statif suivant la revendication 1, caractérisé par le fait que les moyens de couplage (48 à 50) engrènent dans des pièces profilées (52, 53) montées sur le sélecteur sous-table (7) de l'appareil de radiodiagnostic (1).

6. Statif suivant la revendication 1, caractérisé par le fait que les moyens d'accouplement (48 à 50) engrènent dans des pièces profilées (54 à 57) montées sur le châssis (6) de la table de la couchette pour patient (2).

7. Statif suivant la revendication 6, caractérisé par le fait que les moyens d'accouplement (48 à 50) engrènent dans des pièces profilées (55, 57) montées sur une face frontale du châssis (6) de la table.

8. Statif suivant la revendication 2, caractérisé par le fait que le ressort de soutien (38) réalise une légère surcompensation du poids

**O 033 497**

total du chariot de levage (21) et de l'appareil d'enregistrement d'images (4), qui y est fixé et que le chariot de levage peut être bloqué par l'intermédiaire d'un cliquet (43) dans sa position inférieure extrême.

9. Statif suivant la revendication 1, caractérisé par le fait que la position de l'appareil d'enregistrement d'images (4) est définie tant dans sa position dans laquelle la couche d'image (24) est horizontale, que dans la position dans laquelle la couche d'image est verticale, par des butées (28, 35, 36, 37, 40, 41, 43).

10. Statif suivant la revendication 1, caractérisé par le fait qu'au moins un ressort (51) compensant le couple de rotation est associé au dispositif de fixation (23), pouvant pivoter autour d'un axe horizontal (22), pour le basculement de l'appareil d'enregistrement d'images (4).

11. Statif suivant la revendication 1, caractérisé par le fait que la glissière (32) pour le chariot de levage (21) est inclinée de 20° à 70° par rapport à la verticale.

**Claims**

1. A stand (3) for an X-ray imaging device (4) which can be inserted beneath a patient support plate (2) of an X-ray investigation apparatus (1), having a stand carriage (20) which can be moved two-dimensionally over the floor, and a lifting carriage (21) which can be adjusted in height on the stand carriage (20) and which has a support (23) which can be pivoted through 90° about a horizontal axis on the lifting carriage (21) for the imaging device (4) which can be tilted from a position with a horizontal image layer into a position with a vertical image layer, characterised in that a guide path (32) which is inclined to the perpendicular is provided on the stand carriage (20); that the stand carriage (21) has a horizontal platform (34) at one end of which the imaging device (4) is mounted so as to be pivotable by means of the support (23); that a frame element (33) which moves in the guide path (32) and which forms part of the lifting carriage (21) is rigidly connected to the platform (34) and forms an acute angle therewith; that the stand carriage (20), the lifting carriage (21) which moves along the stand carriage, and the support (23) which is pivotally mounted on the lifting carriage, are arranged in their horizontal starting position beneath the imaging device (4), and that coupling means (48, 49, 50) which can be brought into engagement with the X-ray investigation apparatus (1) and which serve for the determinate positioning of the imaging device (4) relative to the X-ray investigation apparatus (1) are connected to the stand carriage.

2. A stand as claimed in Claim 1, characterised in that the overall weight of the lifting carriage (21), the support (23) and the imaging device (4) is compensated by at least one support spring (38) which is built into the stand carriage (20).

3. A stand as claimed in Claim 1, characterised in that stop means (40, 43), which are adjusted to the height of the support surface and the underside of the patient support plate (2) are provided for the upper and lower delimitation of the lifting path of the lifting carriage (21).

4. A stand as claimed in Claim 1, characterised in that at its two longitudinal sides, guide tabs (48, 49, 50) are arranged, which can be inserted into corresponding profiled bars (52 to 57) of the X-ray investigation apparatus, which bars are aligned transversely to the longitudinal axis of the patient support plate (2).

5. A stand as claimed in Claim 1, characterised in that the coupling means (48 to 50) engage into profiled elements (52, 53) arranged on the undertable target device (7) of the X-ray investigation apparatus (1).

6. A stand as claimed in Claim 1, characterised in that the coupling means (48 to 50) engage into profiled elements (54 to 57) arranged on the table frame (6) of the patient support plate (2).

7. A stand as claimed in Claim 6, characterised in that the coupling means (48 to 50) engage into profiled elements (55, 57) arranged at one end of the table frame (6).

8. A stand as claimed in Claim 2, characterised in that the support spring (38) somewhat overcompensates for the overall weight of the lifting carriage (21) and of the imaging device (4) which is attached thereto, and the lifting carriage can be locked in its lowest position by a catch (43).

9. A stand as claimed in Claim 1, characterised in that the position of the imaging device (4) is defined by stop means (28, 35, 36, 37, 40, 41, 43), both in the position with a horizontally-aligned image layer (24) and in the position with a vertically-aligned image layer.

10. A stand as claimed in Claim 1, characterised in that the support (23) which can be pivoted about a horizontal axis (22) and which is provided for the tilting of the imaging device (4) is assigned at least one spring (51) which compensates for the torque.

11. A stand as claimed in Claim 1, characterised in that the guide path (32) for the lifting carriage (21) is inclined by 20° to 70° to the perpendicular.

FIG 1

FIG 2

FIG 4

FIG 3